# EUROPÄISCHE PATENTSCHRIFT

(11) **EP 0 617 933 B1**
(45) Veröffentlichungstag und Bekanntmachung des Hinweises auf die Patenterteilung: **10.11.1999**
(21) Anmeldenummer: 94107864.4
(22) Anmeldetag: 11.09.1989
(51) Int. Cl.: A61F 2/36

(54) **Hüftgelenksprothese**
Hip-joint prosthesis
Prothèse de la hanche

(30) Priorität: 09.09.1988 DE 3830748
(43) Veröffentlichungstag der Anmeldung: 05.10.1994
(62) Teilanmeldung aus: 89909735.6
(73) Patentinhaber: DRAENERT, Klaus, Dr.med., D-81545 München (DE)
(72) Erfinder: DRAENERT, Klaus, Dr.med., D-81545 München (DE)

(56) Entgegenhaltungen:
- EP-A- 0 102 853
- EP-A- 0 128 036
- EP-A- 0 145 641
- EP-A- 0 201 407
- DE-U- 8 705 921
- FR-A- 2 438 469

## Beschreibung

Die Erfindung betrifft eine Hüftgelenksprothese, die vorzugsweise mit Knochenzement, aber auch zementfrei eingesetzt werden kann.

In der orthopädischen Chirurgie sind Gelenkersatzoperationen Standardverfahren der Therapie geworden, gleichwohl können die bisher zum Einsatz kommenden Prothesen und ihre Verankerungsverfahren noch nicht als Lösung aller bei den Operationen auftretender Probleme angesehen werden. So kommt es beispielsweise bei zementierten Gelenkersatzkomponenten sehr oft zur Zerstörung des Zementköchers und zur Auslockerung des Prothese. Es wurden daraufhin eine Reihe von Prothesen entwickelt, die ohne Verwendung von Knochenzement implantiert werden können. Die bekannten zementfrei implantierten Prothesen weisen jedoch ebenfalls noch Nachteile auf. Beispielsweise konnte festgestellt werden, daß zementfreie Implantate in der ersten Zeit nach der Operation Schmerzen verursachen und in der nachfolgenden Zeit ziemlich unkontrollierte Atrophien am Knochen hervorrufen, die zu pathologischen Frakturen Anlaß geben können (s. I.W Brown und P.A. Ring, Osteolytic changes in the upper femoral shaft following porous-coated hip replacement, J. Bone Joint Surg. 67B, S. 218-221). Darüber hinaus hat die Zahl der Lockerungen durch die zementfreien Prothesen keinesfalls abgenommen, sondern zugenommen.

Im Rahmen von Versuchen konnte am Beispiel des Hüftgelenkes und hier speziell an der Femurkomponente gezeigt werden, daß die unzementierten Komponenten eine erhebliche Deformation des Knochens in den Belastungsphasen verursachen. Diese Deformation ist umso größer, je mehr Masse ein Implantat mit sich bringt, bzw. je größer die Deformierbarkeit des individuellen Knochens ist. Auf der anderen Seite ist es für die zementfreien Implantate zwingend, von Beginn an einen schlüssigen Kontakt zum Knochen zu gewährleisten, was zur Entwicklung von formschlüssigen oder anatomisch adaptierten Komponenten geführt hat (s. J. Henssge, Methode zur Entwicklung anatomisch richtiger Implantatkörper, in "Grenzschichtprobleme der Verankerung von Implantaten unter besonderer Berücksichtigung von Endoprothesen", herausgegeben vom M. Jäger, M.H. Hackenbroch, H.J. Refior, Georg Thieme Verlag Stuttgart, New York, 1981). Ein Extrem dieser Entwicklung ist die sogenannte costum made-Prothese, die beispielsweise von Mulier vorgestellt worden ist (J.C. Mulier, Improvements of the Charnely technique of total hip replacement and future developments, Acta Orthop. Belg. 52, Seiten 392 bis 403). Diese Designs führen aber zu besonders schweren Implantaten Die Reaktion des Knochens ließ dementsprechend eine kräftige Deformation erkennen. Deformationen des Knochens führen aber zu Oberflächenverschiebungen und damit zur Knochenresorption, was die Auslockerung der Prothese zur Folge haben kann.

Es wurden auch Versuche unternommen, die Erfahrungen mit anderen Implantaten auf die Prothetik zu übertragen. So wurde beispielsweise das Prinzip der Vorspannung, welches ein Grundprinzip der Osteosynthesetechnik darstellt, mit dem sogenannten press-fit-Prinzip in die Endoprothetik eingebracht (M. E. Müller, Total hip replacement: planning, technique and complications, in: Cruess, R.L. Mitchell, N.S.: Surgical management of degenerative arthritis of the lower limb, Chapter 10, Seiten 91 bis 113, Lea & Febiger, Philadelphia 1975; M.E. Müller und B. Elmiger: Coxarthrose, 10-Jahres-Ergebnisse der sog. Setzholz-Totalprothese, Orthopäde 8, Seiten 73 - 74, 1979; K. Zweymüller , Knochen- und Gelenkersatz mit biokeramischen Endoprothesen, Facultas, Wien, 1978). Auch bei Anwendung des Prinzips der Vorspannung war jedoch wie bei den schweren Prothesenkomponenten eine starke Deformation die hervorstechende Antwort des Lagerknochens.

Die Untersuchung mehr oder weniger gut zementierter Prothesen, welche lange Jahre getragen worden waren, ließ die Überlegenheit zementierter Komponenten erkennen. Ein geringes Remodelling, d.h. ein geringer Umbau des knöchernen Lagers, war Hinweis für eine gleichmäßige Krafteinleitung, und der geschlossene Knochenkontakt war der Beweis für das Fehlen von Relativbewegungen im Interface, was auf eine relativ geringe Deformation des Knochens durch das Implantat schließen läßt (K. Draenert, Histomorphologische Befunde zur gedämpften und ungedämpften Krafteinleitung in das knöcherne Lager, Vereinigung Nordwestdeutscher Orthopäden, 36. Jahrestagung, Hannover, 15. bis 18. Juni 1986).

Der Erfindung liegt somit die Aufgabe zugrunde, eine Prothese (Endoprothese) bzw. ein Implantat zu entwickeln, welches reproduzierbar gute Ergebnisse im Bezug auf die Einheilung und Beanspruchbarkeit erreichen läßt.

Zur Lösung dieser Aufgabe geht die Erfindung von dem Grundgedanken aus, bei der Konstruktion der Prothese die Zusammenhänge zwischen Deformation und Masse des Implantats bzw. der Deformierbarkeit des Knochens zu berücksichtigen. Es konnte im Rahmen der Erfindung gezeigt werden, daß nicht nur die Dämpfung der Krafteinleitung zu einer geringeren Deformation des Knochens führt, sondern daß die Deformation des Knochens sich auch ganz erheblich von Individuum zu Individuum unterscheidet bzw. beeinflußt werden kann, beispielsweise durch die Gestaltung der Prothese durch Anpassung an verschiedene Patientenkonfigurationen beispielsweise durch voneinander unabhängige Verstellbarkeit im Kopf-, Halsteil der Prothese, was in der EP-A-0201407 nicht möglich ist, und durch Aussteifung mit Knochenzement. Die EP-A-0 201 407 offenbest die Merkmale des Oberbegriffs des Anspruchs 1.

Die vorstehende Aufgabe wird durch die Prothese gemäß der Erfindung gelöst. Die erfindungsgemäße Prothese berücksichtigt und löst die Probleme der Deformation des Knochens durch das Implantat und der korrekten Ausrichtung des Prothesenkopfes.

Die erfindungsgemäße Prothese zeichnet sich dadurch aus, daß sie die größtmögliche Oberfläche, die für die Krafteinleitung in die Prothese bzw. in den Knochen zur Verfügung steht, ausnützt und ein Höchstmaß an Rotationsstabilität aufweit. Die Prothese kann mit ihrer Masse der individuellen Deformierbarkeit des Knochens angepaßt werden und kann im Extremfall als Leichthaukonstruktion ausgeführt werden.

Die erfindungsgemäße Prothese läßt sich derart einsetzen, daß nur ein geringer Knochenverlust entsteht. Beim Verfahren zum Einsetzen der Prothese wird zunächst mit Diamanthohlschleifen ein Schlitz gefräst. Die Zementauffüllung erfolgt ebenfalls über mit Diamanthohlschleifen gesetzte Verankerungsstollen unter Beaufschlagung mit einen starken Vakuum. Bei zementfreier Implantation kann die erfindungsgemäße Prothese aufgrund ihrer Konstruktion vorgespannt werden und so porösen oder mit Keramik beschichteten Implantaten für die Zeit der Knochenheilung eine mechanisch stabile Fixation gewahrleisten.

Die erfindungsgemäße Prothese weist vorzugsweise einen Kopfteil, einen Halsteil und den Stiel oder Schaft auf.

Erfindungsgemäß ist der Kopf oder der den Kopf aufnehmende Konus am Halsteil ein-, zwei- oder dreidimensional einstellbar oder verschiebbar angeordnet. Beispielsweise kann ein auf eine Steckschiene aufgesteckter Kopf in der Frontalebene und/oder in der Sagittalebene verstellt werden. Auf diese Weise gelingt es, für verschiedene Konfigurationen von Patienten exakt ein Alignment einzustellen. Unter Alignment versteht man die exakte Ausrichtung des Kopfes in Richtung auf und die Einleitung der Kraft in das Sacroiliacalgelenk, das physiologischerweise die Kraft aufnimmt ung auf die Wirbelsäule überträgt. Die Einstelleinrichtung ist so ausgebildet, daß die Einstellbarkeit in den einzelnen Richtungen unabhängig voneinander gegeben ist, um eine möglichst große Freiheit und Anpaßbarkeit beim Alignment zu gewährleisten.

Der Kopfe kann in jeder beliebigen Stellung, beispielsweise durch Stellschrauben fixiert werden. Der Halsteil kann ebenso in der Länge verstellt werden, wodurch eine exakte Einstellung in die Belastungsebene, beispielsweise aus Trochantermittelpunkt, Rotationszentrum, Symphysenzentrum erreicht wird.

In einer einfachen Ausführungsform weist die Prothese eine konusförmige Aufnahme für einen beliebigen Kugelkopf auf, wie er beispielsweise in Stahl oder Keramik im Handel ist. Die Variabilität des diese Ausführungsform kennzeichenden medialen Aufbaues ist auch bei verschiedenen individuellen Femora gering, so daß die gängigen morphologischen Varianten der Hüftgelenke mit drei bis vier Größen abgedeckt werden können. Die Zahl läßt sich noch weiter verringern, wenn eine variable Aufnahme am Schenkelhals vorgesehen ist, die in der Frontalebene verstellbar ist. Gegebenenfalls ist auch eine zusätzliche Verstellbarkeit in der Sagittalebene und/oder in der Höhe vorgesehen.

In einer Ausführungsform der Erfindung weist die erfindungsgemäße Prothese einen seitlich (lateral) offenen Hohlkörper auf, der sich den kraftübertragenden Flächen anlegt, und stellt ein offenes Hohlkörperimplantat dar, wobei die Steifigkeit der Prothese vorzugsweise von proximal nach distal abnimmt.

Hohlkörperimplantate als solche sind grundsätzlich bereits bekannt. So ist aus der EP-A-190446 ein metallenes Knochenimplantat bekannt, das aus zwei zu einem geschlossenen Hohlkörper zusammengefügten Blechschalen besteht. Durch die Ausbildung als geschlossener Hohlkörper wird zwar das Gewicht des Implantats reduziert; die Steifigkeit des Implantats entspricht aber im wesentlichen herkömmlichen Vollimplantaten und die vorstehend erläuterten Probleme im Zusammenhang mit der Kraftübertragung und der Deformierbarkeit des Knochens werden hierdurch nicht gelöst.

Aus der EP-A-65481 ist eine Hüftgelenksprothese mit einem Hohlschaft bekannt, der durch lochförmige Aussparungen örtliche Materialschwächungen aufweist, durch die der Hohlschaft bezüglich seiner Längs- und/oder Biegesteifigkeit an die Längs- bzw. Biegesteifigkeit des umgebenden Knochenbereichs angepaßt werden soll. Auch dieses Implantat vermag die vorstehend erläuterten Probleme nicht zu lösen.

Ferner ist eine zementfreie, selbstverblockende Hüftgelenkprothese bekannt, bei der aus dem vollen Prothesenschaft Locher ausgeschnitten sind, so daß lediglich dünne Verbindungsstege verbleiben, die den medialen und lateralen Teil der Prothese verbinden (S. Tepic, S.M. Perren, Cementless self-locking stem for hip prosthesis).

Demgegenüber weist die erfindungsgemäße Prothese in einer Ausführungsform seitlich entlang ihres Stiels und gegebenenfalls ihres Halsteils eine durchgehende Öffnung oder Rinne in Form eines langgezogenen Schlitzes auf, der sich über den wesentlichen, Teil der Länge des Implantats, vorzugsweise über mehr als 80 % oder gegebenenfalls über mehr als 90 % der Länge des Implantats erstreckt. In diesem Bereich weist die Prothese einen U-förmigen oder hufeisenförmigen Querschnitt auf, wobei der Steg des U bzw. des Hufeisens den medialen Teil der Prothese bildet und die Enden dem Arme des U abgerundet sind. Die Prothese ist dort offen, wo keine oder nur geringe Kraftübertragung erfolgen muß. Die Prothese stellt deshalb die größtmögliche Oberfläche für die Kraftübertragung bei gleichzeitiger Verringerung der Masse zur Verfügung und kann in verschidener Weise den Bedürfnissen des Patienten angepaßt werden. Die Tiefe der Rinne kann bis zu 90 % der Ausdehnung der Prothese in ihrer Medial-Lateral-Richtung betragen, vorzugsweise etwa 30 bis 80 %, besonders bevorzugt etwa 40 bis 70 %, und kann in Längsrichtung der Prothese variieren. Die Rinne kann im Rahmen der Erfindung auch sehr flach und wenig ausgeprägt sein und kann im Extremfall auch ganz verschwinden, so daß die Prothese als Vollimplantat ausgebildet ist, falls durch das Prothesendesign, insbesondere deren Massenverteilung, sichergestellt ist, daß die zugrundeliegende Aufgabe gelöst wird.

Der Kopfteil ist vorzugsweise als Halbschale ausgebildet, welche lediglich das tragende Dach des Femurkopfes bedeckt, da nur dort eine Kraftübertragung erfolgt. Um der Schreitbewegung Rechnung zu tragen, ist vorzugsweise vorne (ventral) und hinten (dorsal) die Gleitfläche tiefer heruntergezogen.

Der Kopfteil kann auch als Vollersatz ausgebildet sein, der einem Konus im Halsteil aufgesetzt wird oder selbst mit einem Konus versehen ist und in den Halsteil eingesteckt werden kann. Der Kopfersatz kann auch einem ausgezogenen und vorzugsweise massiv ausgebildeten Plateau des Halsteiles direkt aufgeschweißt oder aufgesteckt werden.

Der Halsteil der Prothese ist massiver und gegebenenfalls als Vollkörper ausgebildet und stellt den Übergang zur im Querschnitt vorzugsweise U-förmigen Rinne des Stieles dar, dient aber auch gegebenenfalls zur Aufnahme eines Gelenkes, als Gleitschiene oder als Steckgelenk, wenn eine modulare Implantatkomponente eingesetzt werden soll. Bei dieser Ausführungsform ist es auch möglich, die Halbschale der tragenden Fläche gegen einen konventionellen Steckkonus auszutauschen. Sofern noch ein Teil des Knochens vom Schenkelhalskopf erhalten ist, ist es auch möglich, einen entsprechend modifizierten Kopf aufzuschieben, wodurch eine wesentlich größere kraftaufnehmende Fläche am Schenkelhals erhalten bleibt. Die Prothese kann aber auch einteilig aus einem Stück hergestellt sein; der Halsteil liegt dann an den starken Strukturen des Schenkelhalses lateral auf.

Der Stiel ist in seinem Querschnitt vorzugsweise als offenes Halbrohr oder entlang seines Umfangs zu etwa der Hälfte bis 3/4 geschlossenes Rohr, vorzugsweise als zu 2/3 bis 3/4 geschlossenes Rohr ausgebildet, das zur Prothesenspitze hin konisch zuläuft. Es ist wesentlich, daß der Stiel exakt entlang der durch Verlängerung der freien Markhöhle gebildeten Achse lateral eingeführt wird, ohne daß dadurch die Schenkelhalsstrukturen unnötigerweise verletzt werden Das Halbrohre oder die Hohlrinne legt sich mit ihrem im Querschnitt konvexen Teil der starken Knochenstruktur des proximalen Femurquerschnittes an.

Bei einer Ausführungsform der Erfindung ist die Massenverteilung in jeder Querschnittsfläche zumindest im proximalen Abschnitt so gestaltet, daß die Hauptmasse dorsal und medial ausgerichtet ist. Dies beruht darauf, daß systematische Untersuchungen anhand von Röntgenbildern gezeigt haben, daß in diesen Bereichen die hauptsächliche Kraftübertragung erfolgt. Wegen dieser Asymmetrie ist in der Regel eine Rechtsversion und eine Linksversion der Prothese erforderlich.

In Richtung auf das distale Ende der Prothese nimmt die mediale Ausrichtung der Masse allmählich ab, und in der distalen Prothesenhälfte ist die mediale und laterale Massenverteilung bezüglich der Achse im wesentlichen symmetrisch. Die dorsale Ausrichtung der Prothesenmasse nimmt in Richtung auf das distale Ende der Prothese rasch ab, und in der distalen Prothesenhälfte ist die Masse vorwiegend ventral ausgerichtet. In der Seitenansicht betrachtet ist die dorsal-ventrale Massenverteilung im wesentlichen S-förmig ausgebildet: der in proximalen Bereich vorliegende dorsale Massenüberschuß geht im mittleren Prothesenabschnitt gegen Null und wird im distalen Prothesenabschnitt negative, d.h. es liegt ein ventraler Massenüberschuß vor, der ar der Prothesenspitze wieder zu Null wird. Dennoch ist die Prothese als Geradschaftprothese zu bezeichnen, da die beispielsweise zwischen dem Einschlagpunkt der Prothese und der Prothesenspitze verlaufende Prothesenachse durchgehend innerhalb des Prothesenkörpers verläuft. Durch die genannte Massenverteilung ist die Prothese in allen Schnittebenen optimal an die Form des Oberschenkelschafts angepaßt und bietet einen guten Sitz.

Die mediale Längskante der Prothese weist in ihrem proximalen Abschnitt eine konkave Krümmung auf. Die Krümmung ist stärker als bei herkömmlichen Prothesen, und der Krümmungsradius beträgt vorzugsweise weniger als 10 cm oder weniger als 8 cm, besonders bevorzugt etwa 4 bis 6 cm. Mit diesem längskonkaven Anteil legt sich die Prothese exakt der kraftaufnehmenden Struktur des medialen Femuranteils an. Die Massenverteilung, d.h. die Dicke der Prothese im Längsschnitt, ist entlang dieser konkaven Oberfläche vorzugsweise so ausgebildet, daß die Hauptmasse proximal, d.h. kopfnahe verteilt ist und von hier aus vorzugsweise kontinuierlich abnimmt bis zur Spitze. Dies beruht darauf, daß Versuche gezeigt haben, daß die Krafteinleitung proximal konzentriert ist, so daß in diesem Bereich auch die Prothesenmasse konzentriert sein muß. Der gesamte Massenschwerpunkt der Prothese liegt vorzugsweise innerhalb des Knochens, um unerwünschte Kippbewegungen und Drehmomente zu verhindern. Besonders bevorzugt liegt der Massenschwerpunkt im proximalen Prothesenabschnitt im medialen und dorsalen Teil der Prothese, er kann gegebenenfalls aber auch mittig zwischen dorsal und ventral liegen. Aufgrund der Massenabnahme nimmt auch die Steifigkeit der Prothese von proximal nach distal ab.

Untersuchungen haben gezeigt, daß in der "heel strike"-Phase, d.h. beim Aufsetzen der Ferse, ein hohem Retrotorsionsmoment auftritt, d.h. daß der Kopf nach hinten gedreht wird, wogegen wahrend der Schwingungsphase lediglich ein geringeres Antetorsionsmoment auftritt. Um eine hohe Rotationsstabilität zu erreichen, müssen diese Momente durch die Ausladungen oder "Arme" der im Querschnitt hufeisenförmigen Prothese aufgefangen werden. Besondere Aufmerksamkeit ist der Ausladung der Hohlrinne im proximalen Bereich zu schenken. Der Stiel der Prothese kann hier flügelartige Ausladungen aufweisen, wodurch eine sehr hohe Rotationsstabilität erreicht wird. Die Flügel können bei Rechts-Links-Prothesen auch asymmetrisch ausgebildet sein. In diesem Falle wird vorzugsweise der dorsale Flügel oder Arm verbreitert und gegebenenfalls auch verlängert, da die Hauptrotationskomponente, wie vorstehend erläutert, eine Retrotorsion der Prothese darstellt und zwar beginnend beim Aufsetzen der Ferse bis zur Standbeinphase. Durch die Verkürzung des ventralen Arms kann außerdem eine Materialersparnis ohne Stabilitätsverlust erzielt werden.

Durch verschiedene Variationen in der Dicke des Stiels kann im Rahmen eines Biopsieprogrammes die in Bezug auf die Deformation der Prothese günstigste und stabilste Version bestimmt werden. Dabei wird zunächst eine beispielsweise zylinderförmige Knochenbiopsie entnommen und die individuelle Deformierbarkeit des Knochens gemessen. Danach wird mittels eines kalibrierbaren Röntgenkontrastverfahrens die Knochendichte bestimmt. Aus der individuellen Deformierbarkeit und der Knochendichte wird die gesamte Deformierbarkeit des Knochens bestimmt. Bei niedriger Deformierbarkeit kann gegebenenfalls eine zementfreie Prothese verwendet werden. Bei hoher Deformierbarkeit ist eine zementierte Prothese bevorzugt.

An Stellen hoher Krafteinleitung, also insbesondere proximal, muß die Prothese eine höhere Wandstärke aufweisen und auch der Knochenzement muß möglichst dick sein, um eine gleichmäßige Krafteinleitung ohne unerwünschte Deformation des Knochens zu ermöglichen

Die Spitze der Prothese ist so gestaltet, daß sie vor allem nach ventral, aber auch nach lateral breite Auflageflächen bietet. An keiner Stelle sind Kanten oder Grate ausgebildet, die Anlaß zu Streßkonzentrationen und damit zu Streßfrakturen des Zementköchers geben könnten.

Die Bestimmung der Prothesengrößen orientiert sich vorzugsweise an der Distanz der Femurschaftachse in der Ebene des Trochanter minors zur tragenden Struktur medial am Femur.

Die Grundachse oder Säule der Konstruktion stellt die Femurschaftachse dar. Der Schaft oder Stiel der Prothese ist so konstruiert, daß er entlang dieser Achse geführt wird und stabil fixiert sitzt. Dies kann einerseits durch die Länge des Stieles und andererseits auch durch das vorstehend erläuterte Profil des Querschnittes erreicht werden.

Die Prothese kann auch so konstruiert werden, daß mehrere Komponenten übereinander an einer hohlen oder rinnenförmigen Achse aufgereiht werden, wodurch ein Modularsystem geschaffen werden kann.

Die Länge der Prothese kann variabel gestaltet werden und beträgt in der Regel zwischen 13 und 22 cm. Die Länge ist besonders variabel zu gestalten, wenn die Prothese als Modularsystem aufgebaut ist.

Das modulare Baukastensystem, bei dem ein halboffenes Rohr bzw. eine Hohlrinne als Achse die modularen Komponenten aufnimmt, bietet für Reoperationen entscheidende Vorteile, da die Komponenten einzeln ausgetauscht werden können.

Für die zementfreie Implantation kann die Prothese auch vorgespannt eingesetzt werden. Dies ist besonders bevorzugt wenn gewährleistet werden soll, daß poröse oder spongiosaartig strukturierte Oberflächen knöchern integriert werden sollen und Relativbewegungen von Oberflächen vermieden werden müssen. Zu diesem Zweck wird die Prothese über den Kopf verhakt und distal vorgespannt oder es wird umgekehrt verfahren. Es zeigt sich zwar, daß diese Vorspannung im Verlauf der folgenden drei Monate zum größten Teil abgebaut wird, in dieser Zeit kann jedoch eine knöcherne Überbrückung erfol

Die Prothese kann aus einer gängigen Co-Cr-Mo-Legierung gegossen oder geschmiedet werden. Sie kann auch aus Titan oder einer Ti-Legierung gefertigt werden. Es ist auch möglich, daß die Prothese zur Erhöhung der Oberfläche nach Art der Spongiosametalle durchgehend porös ist oder daß sie mit einer porösen Beschichtung oder einer Keramik-, Bioglas- oder Apatitbeschichtung hergestellt wird.

Der Stiel der Prothese kann vorzugsweise auch aus geschichteten Gittern geformt werden, die punktuell oder über Tragrippen verschweißt werden.

Die Erfindung wird nachstehend anhand der Zeichnung näher erläutert. Es zeigen:
- Figur 1: eine Ausführungsform der erfindungsgemäßen, im Femur implantierten Prothese, wodurch zur näheren Erläuterung der Querschnitt der Prothese im proximalen Abschnitt schematisch eingezeichnet ist,
- Fig. 1a bis 1c: typische Prothesenquerschnitte, z.B. im proximalen Bereich des Stiels,
- Fig. 2a bis 2b: verschiedene Ausführungsformen der Verschiebbarkeit des Steckkonus,
- Fig 3a und 3b: eine weitere Ausführungsform der erfindungsgemäßen Prothese in Vorder- und Seitenansicht,
- Figur 4: eine Ausführungsform der erfindungsgemäßen, als Modularsystem aufgebauten Prothese, und
- Fig. 5a bis 5d: verschiedene Phasen bei der Verankerung der erfindungsgemäßen Prothese.

In den Zeichnungen sind die Prothesen, falls nicht anders angegeben, jeweils als (im implantierten Zustand) Vorderansicht gezeigt.

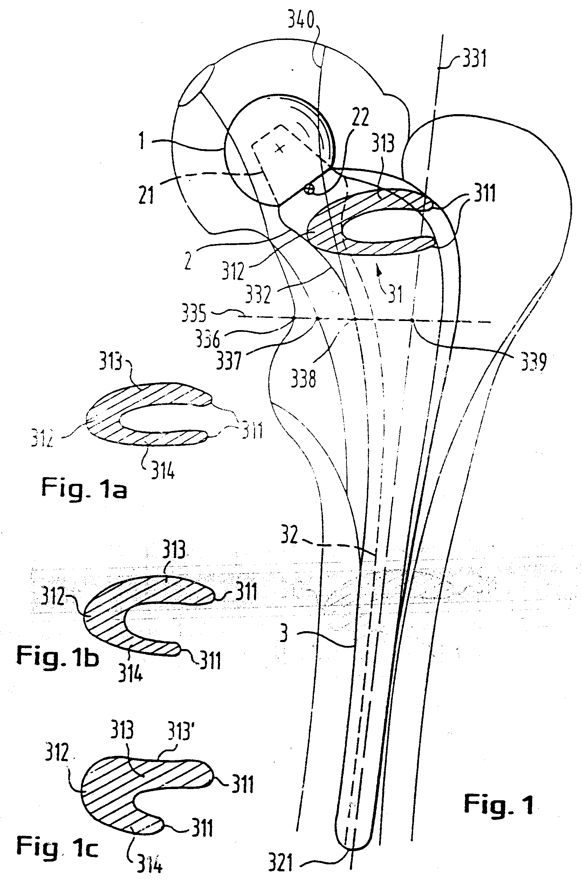

Die schematisch im Oberschenkelknochen dargestellte Prothese gemäß Figur 1 weist einen aufsteckbaren Kopf 1 aus Keramik oder Stahl auf, der auf dem konusförmig ausgebildeten Teil 21 des Halses 2 der Prothese aufsitzt, wobei der Winkel zur Schaftachse zwischen 120 und 150° beträgt. Dieser Winkel ist über eine Achse 22 einstellbar und fest zu fixieren, oder er ist in der Konstruktion vorgegeben, in der Regel als 140°.

Der Halsteil 2 ist fest mit dem Stiel 3 verschweißt. Der Stiel 3 weist in Längsrichtung medial eine konkave Krümmung 332 auf, die sich den auf das tragende Dach gerichteten tragenden Strukturen medial im Femur anpaßt. Diese Strukturen lassen sich beispielsweise durch Röntgenaufnahmen sichtbar machen. Nach dem Wolff'schen Gesetz richtet sich der Knochen nach der Beanspruchung aus und paßt sich dieser an. Aus der Richtung der tragenden Strukturen bzw. Trabekel läßt sich somit die Richtung der Beanspruchung bzw. die Richtung der Krafteinleitung ermitteln. Dem trägt die Ausbildung der konkaven Krümmung 332 Rechnung.

Im Längsschnitt, der in Figur 1 schematisch dargestellt ist, siehe die gestrichelte Linie 32, wobei der Prothesenteil medial der Linie 32 massiv ausgebildet ist, weist die Prothese einen massiven Halsteil und eine nach distal abnehmende Schichtdicke bzw. Wandstärke auf. Die Spitze 321 der Prothese ist rund gestaltet.

Die Größe der Prothese wird anhand des Röntgenbildes mit Hilfe der vorzugsweise durch den Einschlagpunkt der Prothese verlaufenden Schaftachse 331 und der gestrichelt eingezeichneten Trochanter-minor-Schnittebene 335 bestimmt. Die Trochanter-minor-Schnittebene 335 ergibt sich aus der horizontalen Beanspruchungsebene im Kniegelenk und ist in der Regel um etwa 81° zur Schaftachse geneigt. Der Punkt 339 stellt den Schnitt der Trochanter-minor-Schnittebene 335 mit der Schaftachse 331 dar, der Punkt 338 den Schnitt der Trochanter-minor-Schnittebene 335 mit der Linie 340 der Krafteinleitung bzw. der Projektionslinie der tragenden Struktur, der Punkt 337 den Schnitt der Trochanter-minor-Schnittebene mit der inneren (endostalen) Knochenrinde bzw. dem Markhöhlenrand, und der Punkt 336 den Schnitt der Trochanter-minor-Schnittebene 335 mit dem Knochen an der Einsenkung am Übergang zum Trochanter minor, wo der Muskelansatz liegt. Die zu verwendende Prothesengröße ergibt sich entweder direkt aus der Strecke zwischen den Punkten 339 und 338 oder wird indirekt aus der Distanz zwischen den Punkten 339 und 337 bzw. den Punkten 339 und 336 bestimmt.

Der Querschnitt des Stieles, der schematisch beispielsweise als 31 eingezeichnet ist, ist vorzugsweise ellipsenförmig, parabelförmig, U-förmig oder hufeisenförmig uni kann gegebenenfalls auch ein (dreiblattriges) Kleeblattprofil aufweisen. Die Enden 311 der Arme des U bzw. des Hufeisens sind stumpf abgerundet. Die Massenverteilung bzw. die Wandstärke des Querschnitts ist vorzugsweise exzentrisch, wobei medial (bei 312) und dorsal (bei 313) Verdickungen vorgesehen sind, da dort hauptsächlich die Kraftübertragung erfolgt.

Bei dem in Figur 1a dargestellten typischen Prothesenquerschnitt im proximalen Bereich sind der dorsale Arm oder Flügel 313 und der ventrale Arm oder Flügel 314 des Prothesenquerschnitts im wesentlichen gleich lang ausgebildet, während bei der Ausführungsform gemäß Figur 1b der ventrale Arm oder Flügel 314 kürzer als der dorsale Arm oder Flügel 313 ist. Dies führt zu einer weiteren Materialersparnis ohne wesentliche Stabilitätseinbußen, da die Krafteinleitung vorwiegend im medialen und dorsalen Bereich erfolgt.

Bei der Ausführungsform des Querschnitts gemäß Fig. 1c weist der dorsale Arm 313 an seiner Außenseite eine konkave Einbuchtung 313' auf. Hierdurch wird die Rotationsstabilität weiter verbessert.

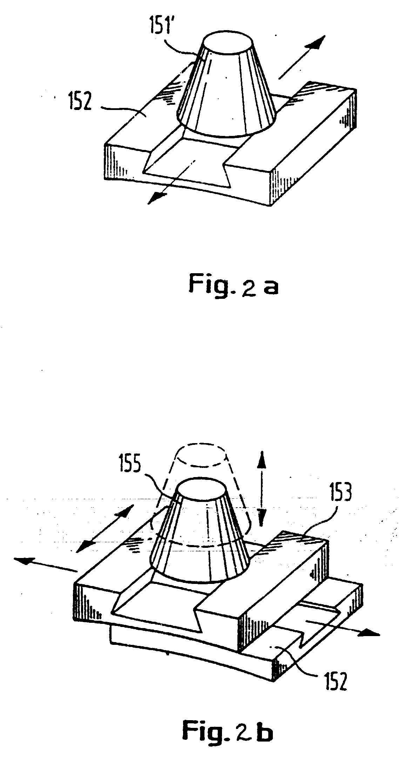

In den Figuren 2a und 2b ist dargestellt, daß das Halsteil so ausgebildet sein kann, daß der Kopf in ein, zwei oder drei Ebenen verstellbar und exakt einstellbar ist.

Gemäß Figur 2a ist ein Steckkonus 151', auf den der Kopf aufgesteckt wird, auf einer Schiene 152, welche mit dem massiven Halsteil der Prothese fest verschweißt ist, in der Frontalebene (parallel zur Stirn) in Richtung der Pfeile verschiebbar. Die Schiene kann beispielsweise als Schwalbenschwarzführung ausgebildet sein.

Anstelle der Ausführungsform gemäß Figur 2a kann die Schiene auch gekrümmt sein, und der Steckkonus kann ferner in der Sagittalebene (senkrecht zur Stirn) kippbar sein, vorzugsweise um 5 bis 10° und ebenfalls nach dorsal.

Gemäß Figur 2b ist der Steckkonus 155 für einen Kopf oder eine Schale mittels zweier zueinander senkrechter Schienen 152 und 153 sowohl in der Frontalebene als auch in der Sagittalebene verschiebbar bzw. kippbar und ist außerdem in Vertikalrichtung beweglich (vgl. die gestrichelt eingezeichnete Stellung). Der Konus ist somit, wie durch die Pfeile angedeutet, in drei Ebenen verstellbar und beispielsweise mittels Schrauben in jeder Position fest fixierbar.

Neben den in den Figuren 2a und 2b lediglich schematisch gezeigten Ausfüßrungsformen können auch noch andere Mittel gewählt werden, um der Kopfteil der Prothese in ein, zwei oder drei Richtungen bzw. Ebenen verstellbar zu machen, um ein perfektes Alignment zu gewährleisten.

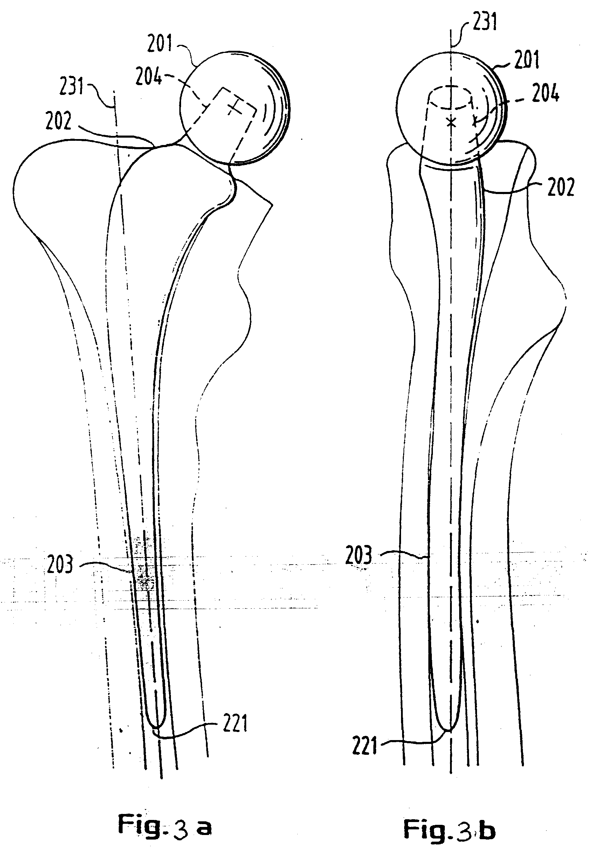

Die Ausführungsform der Prothese gemäß Figur 3, die in Figur 3a in Vorderansicht und in Figur 3b in Seitenansicht dargestellt ist, ist massiv und zeigt somit keine Ausführungsform der Erfindung. Die Prothese weist in Seitenanischt eine S-förmige Massenverteilung auf, sie ist jedoch dennoch eine Geradschaftprothese, weil die Prothesenachse durchgehend innerhalb des Prothesenkörpers verläuft.

Die Prothese gemäß Figur 3 weist ebenfalls einen Kopf 201, einen Hals 202 und einen Schaft 203 auf. Der Kopf 201 sitztauf einem konusförmigen Teil 204 des Halses 202 auf. Die Prothesenachse ist mit 231 bezeichnet. Aus der Vorderansicht gemäß Figur 3a ist ersichtlich, daß der Massenschwerpunkt im proximalen Abschnitt der Prothese weit im medialen Bereich liegt, und sich in Richtung auf das distale Ende der Prothese allmählich an die Achse annähert. Im distalen Prothesendrittel ist die Massenverteilung der Prothese medial und lateral im wesentlichen symmetrisch.

Aus der Seitenansicht gemäß Figur 3b ist ersichtlich, daß der Massenschwerpunkt im Prothesenhals 202 und im proximalen Teil des Prothesenschafts 203 weit dorsal liegt, daß die Prothese dann aber in Richtung auf das distale Ende S-förmig geschwungen ist. Etwa in der Prothesenmitte ist die Massenverteilung dorsal und ventral nahezu symmetrisch, während in der unteren Prothesenhälfte der Massenschwerpunkt ventral liegt. Etwa 1/5 bis 1/6 der Prothesenlänge von der Prothesenspitze 221 entfernt liegt der Massenschwerpunkt am weitesten ventral, während er sich zur Prothesenspitze 221 hin wieder auf die Achse 231 der Prothese zu bewegt und in der Nähe der Prothesenspitze 221 nahezu symmetrisch liegt. Durch die Ausgestaltung der Prothese gemäß Figur 3 ist eine ausgezeichnete Anpassung an den Knochenkanal gegeben, und die Prothese kann trotz ihrer in Seitenansicht S-förmigen Ausbildung dennoch wie eine Geradschaftprothese in den Knochen eingeschlagen werden, da die S-Form so gewählt ist, daß die Prothesenachse 231 durchgehend innerhalb des Prothesenkörpers verläuft.

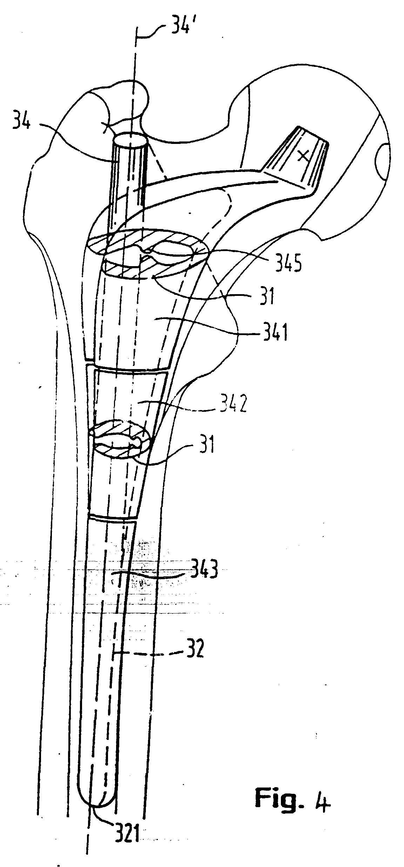

Figur 4 zeigt schematisch eine als Modularsystem aufgebaute Prothese. Diese besteht aus einer lateral, d.h. seitlichkörperfern in der Markhöhlenachse 34' angeordneten Achse 34 und aufsteckbaren medialen Komponenten 341, 342 und 343. Wie anhand der Komponente 341 dargestellt, weisen die Komponenten eine die Achse 34 umfassende Führung 345 auf, deren Form an die Achse 34 angepaßt ist, und sind im übrigen ebenfalls als im Querschnitt hufeisenförmige Hohlkörper ausgebildet (vgl. die Querschnitte 31).

### Beispiel

Die Figuren 5a bis 5 zeigen verschiedene Phasen des Einsetzens einer Prothese.

Bei einem Patienten mit fortgeschritten arthrotisch verändertem Hüftgelenk soll ein künstliches Gelenk eingesetzt werden. Nach Anfertigen von zwei Röntgenbildern des Hüftgelenkes (anterior-posterior und axial) mit eingeblendetem Maßstab wird ein endoprothetischer Ersatz geplant. Nach Auswahl der entsprechenden Prothese und den üblichen Operationsvorbereitungen wird der Patient operiert und das erkrankte Hüftgelenk dargestellt. Nach Eröffnung und Abtragen der Gelenkkapsel wird das runde Kopfband durchtrennt und der Hüftkopf luxiert. Nach Entfernen der restlichen Kapsel wird die Markhöhle in ihrer geraden Verlängerung zwischen Trochanter maior (großer Rollhügel) und dem Schenkelhals mit einer Diamanthohlschleife, beispielsweise gemäß EP-A-99 371, in der Weise eröffnet, daß nach den Naßschleifen eines ca. 14 mm messenden Ringdefektes mit einer Breite von ca. 500 mm der kortiko-spongiöse Knochenzylinder mit einem speziellen Extraktor, beispielsweise ebenfalls gemäß EP-A-99 371, entnommen wird und im Anschluß daran mit einer langen Zylinderhohlschleife im Naßschleifverfahren die Markhöhle vorbereitet wird (siehe Figur 5a mit der Zylinderhohlschleife 25).

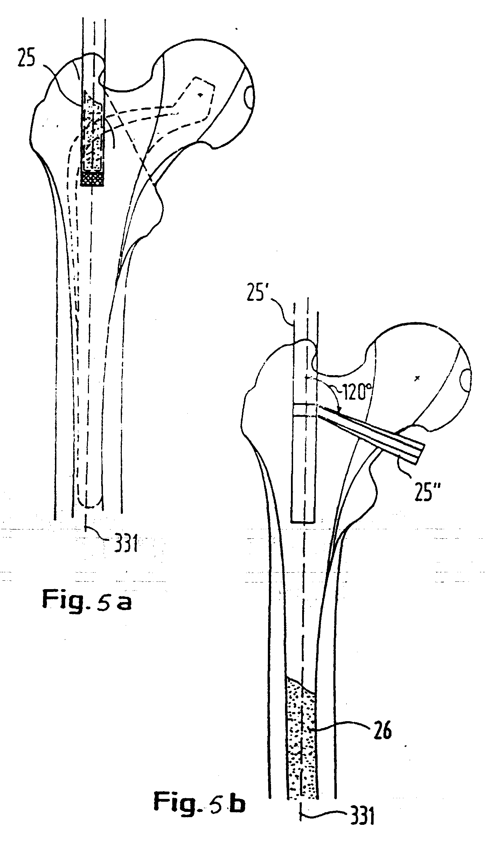

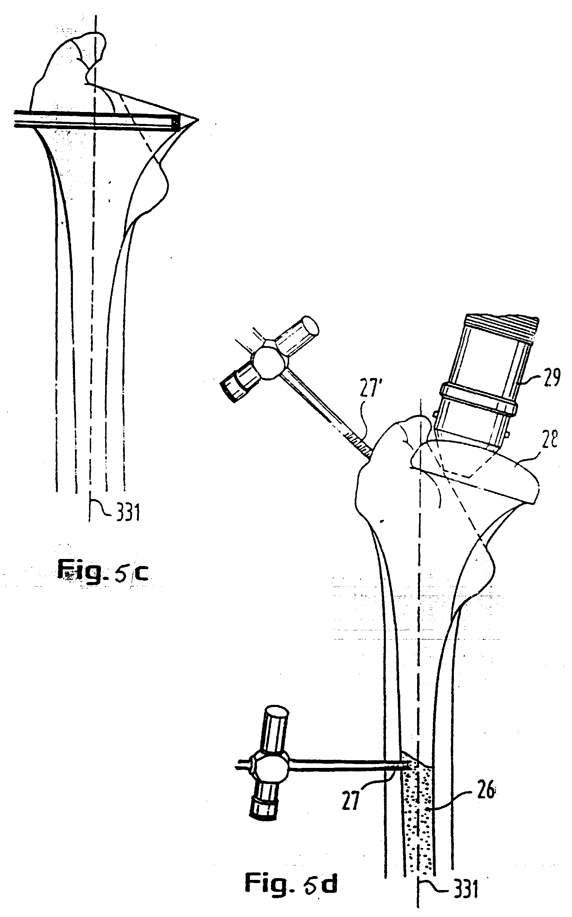

Nach der Drucklavage des knöchernen Lagers wird ein Führungsinstrument 25' eingesetzt, welches mittels einer Schablone 25'' die exakte Schnittebene für das Absetzen des Schenkelhalskopfes vorgibt (siehe Figur 5b). Distal ist die Markhöhle mit einem Plug 26 abgeschlossen. Nach der Resektion des Kopfes wird mit einer im Durchmesser 10 mm messenden Diamanthohlschleife in der Horizontalebene des Calcar femoris (medialer Schnittrand des Schenkelhalses) ein Querstollen in frontaler Richtung von lateral nach medial gefräst (siehe Figur 5c). Der Defekt wird nach außen mit einem verblockenden, jedoch kürzeren Knochenzylinder wieder verschlossen.

Nunmehr wird der zur Eröffnung der Markhöhle entnommene Zylinder gewaschen und wieder eingesetzt und mit einem Kunststoffstössel nach distal verschoben. 2 cm unterhalb der geplanten Prothesenspitze wird die Markhöhle nach Stichincision und Vorschieben eines Troikas anterolateral eröffnet und eine selbstschneidende Kanüle 27, beispielsweise gemaß EP-A-305 417, in die Kompakta eingeschraubt. Durch die Kanüle wird ein Kirschnerdraht eingeschoben. Der Knochenzylinder wird von proximal soweit vorgeschoben, bis der Kirschnerdraht wackelt. Dieser wird daraufhin zurückgezogen und der Plug noch 2 cm vorgeschoben und die Kanüle drei Umdrehungen weiter vorgedreht. Das in dieser Weise vorbereitete Knochenbett nimmt zunächst die Probeprothese auf, mit der der Prothesensitz getestet wird. Sobald die Probeprothese ideal sitzt und sich der medialen Spongiosa großflächig anlegt, wird sie wieder herausgenommen. Nunmehr wird in die dorsale Hälfte des Trochantermassives ebenfalls eine Drainagekanüle 27' eingeschraubt und beide Kanülen werden an eine Vakuumpumpe angeschlossen, welche jedoch noch nicht eingeschaltet ist. Der Schlauch zur proximalen Kanüle bleibt zunächst abgeklemmt.

Die Markhöhle wird nun mit einem Silikonaufsatz 28 versiegelt, die Knochenzementspritze 29 wird aufgesetzt und die Vakuumpumpe eingestellt (siehe Figur 5d). Der Zement wird dadurch gleichmäßig in ein bluttrockenes Bett eingesaugt und füllt die Spongiosawaben im tragenden Bereich aus. Nach dem Absetzen der Zementspritze wird die Prothese langsam eingeschoben, bis sie in der vorher bestimmten Weise sitzt. Nach Aushärten des kaltpolymerisierenden Knochenzementes wird die Pfanne eingesetzt und die Operation wird nach Reposition der Femurkomponente und nach Verschluß der Wunde in Schichten beendet.

## Patentansprüche

1. Hüftgelenkendoprothese mit einem Stiel (3) und einem Kopfteil (1), wobei der Kopfteil (1) der Prothese gegenüber dem Stiel (3) ein-, zwei- oder dreidimensional verstellbar angeordnet ist, dadurch gekennzeichnet, dass die Verstellbarkeit ohne die Notwendigkeit des Austauschs von Prothesenteilen möglich und in den einzelnen Richtungen unabhängig voneinander ist.

2. Prothese nach Anspruch 1, wobei der Kopfteil (1) in zwei senkrecht zueinander stehenden Ebenen, der Frontal- und der Sagittalebene verschiebbar ist.

3. Prothese nach einem der Ansprüche 1 bis 2, wobei der Kopfteil (1) auf einen Konus aufsteckbar ist, welcher auf einer Schiene in der Frontal- und/oder Sagittalebene verschiebbar ist.

4. Prothese nach Anspruch 3, wobei der Konus in der Frontal- und Sagittalebene kippbar ist.

5. Prothese nach den Ansprüchen 3 oder 4, wobei der den Kopfteil (1) tragende Konus mittels zweier Schienen in zwei zueinander senkrechtstehenden Ebenen, der Frontal- und Sagittalebene verschiebbar ist.

6. Prothese nach Anspruch 5, wobei der Konus (21) entlang seiner Konuslängsachse beweglich ist.

7. Prothese nach einem der Ansprüche 1 bis 6, wobei die Prothese als Geradschaftprothese ausgebildet ist, die in ihrer Seitenansicht eine S-förmige Massenverteilung aufweist, wodurch der Massenschwerpunkt der Prothesenquerschnitte im proximalen Prothesenabschnitt dorsal und im distalen Prothesenabschnitt ventral von der Prothesenachse angeordnet ist.

8. Prothese nach einem der Ansprüche 1 bis 7, wobei der Querschnitt der Prothese zumindest in ihrem proximalen Abschnitt im dorsalen Bereich eine konkave Wölbung aufweist.

9. Prothese nach einem der Ansprüche 1 bis 8, wobei die Prothese modular aus mehreren Komponenten aufgebaut ist, welche vorzugsweise mittels einer Führung auf eine Achse aufsteckbar sind, wobei die Komponenten vorzugsweise mindestens teilweise als seitlich offene Hohlkörper ausgebildet sind.

10. Prothese nach den Ansprüchen 1 bis 9, so beschaffen, dass sie als Hüftgelenkersatz in das Femur implantierbar ist.

## Claims

1. A hip joint endoprosthesis having a pedicle (3) and a head piece (1), wherein said head piece (1) of said prosthesis being located one, two and three-dimensionally adjustable in relation to said pedicle (3), characterized in that said adjustment is possible without the necessity to exchange parts of the prosthesis and in that it is independent in each of the respective directions.

2. The prosthesis according to claim 1, wherein the head piece (1) is slideable in two perpendicular planes, namely the frontal and sagittal plane.

3. The prosthesis according to claim 1 or 2, wherein said head piece (1) may be put on a cone which is slideable on a rail in the frontal and/or sagittal plane.

4. The prosthesis according to claim 3, wherein said cone may be tilted in said frontal and sagittal planes.

5. The prosthesis according to claim 3 or 4, wherein said cone carrying said head piece (1) is slideable by means of two rails within two perpendicular planes, namely said frontal and sagittal planes.

6. The prosthesis according to claim 5, wherein said cone (21) is movable along its longitudinal cone axis.

7. The prosthesis according to claims 1 to 6, wherein said prosthesis is formed as straight prosthesis which in side view includes an s-shaped distribution of mass, whereby the center of mass of the sections of said prosthesis is located dorsally in the proximal section of said prosthesis and ventrally to said axis of the prosthesis in the distal section of said prosthesis.

8. The prosthesis according to claims 1 to 7, wherein said cross section of said prosthesis being dished at least in its proximal section in the dorsal portion.

9. The prosthesis according to claims 1 to 8, wherein said prosthesis is composed of several components which preferably can be put onto an axis by means of a guide means, the components at least partially being formed as open hollow bodies with open sides.

10. The prosthesis according to claims 1 to 9, wherein said prosthesis being adapted to be implanted into the femur as hip joint substitution.

## Revendications

1. Endoprothèse pour l'articulation de la hanche comportant une tige (3) et une calotte (1), la calotte (1) étant disposée par rapport à la tige (3) de sorte à ce qu'elle soit réglable selon une, deux ou trois dimensions, caractérisée par le fait que ce réglage est possible sans qu'il soit nécessaire de changer les éléments de la prothèse et qu'il peut s'effectuer dans les différentes directions indépendamment l'une de l'autre.

2. Prothèse selon revendication 1, la calotte (1) pouvant être déplacée dans deux plans perpendiculaires l'un à l'autre, le plan frontal et le plan sagittal.

3. Prothèse selon l'une des revendications 1 à 2, la calotte (1) pouvant être installée sur un cône qui peut se déplacer sur un rail dans le plan frontal et/ou dans le plan sagittal.

4. Prothèse selon revendication 3, le cône pouvant être basculé dans le plan frontal et dans le plan sagittal.

5. Prothèse selon les revendications 3 ou 4, le cône qui supporte la calotte (1) pouvant être déplacé dans le plan frontal et dans le plan sagittal au moyen de deux rails se trouvant dans deux plans perpendiculaires l'un à l'autre.

6. Prothèse selon revendication 5, le cône (21) étant mobilisable selon son axe longitudinal.

7. Prothèse selon l'une des revendications 1 à 6, la prothèse étant réalisée en tant que prothèse à tige droite qui présente vue de côté une répartition de la masse en S, ce qui place le centre de gravité de la masse des sections de la prothèse par rapport à l'axe de la prothèse de manière dorsale pour la portion proximale de la prothèse et de manière ventrale pour sa portion distale.

8. Prothèse selon l'une des revendications 1 à 7, la section de la prothèse présentant au moins dans sa portion proximale une concavité dans la région dorsale.

9. Prothèse selon l'une des revendications 1 à 8, la prothèse modulaire comportant plusieurs composants qui peuvent être emboîtés sur un axe, de préférence au moyen d'un guidage, les composants étant de préférence et au moins partiellement réalisés en tant que corps creux à ouverture latérale.

10. Prothèse selon l'une des revendications 1 à 9, faite en sorte à pouvoir être implantée dans le fémur en tant qu'articulation de la hanche de remplacement.
